# EUROPÄISCHE PATENTANMELDUNG

(11) **EP 1 270 683 A2**
(43) Veröffentlichungstag der Anmeldung: **02.01.2003**
(21) Anmeldenummer: 02011547.3
(22) Anmeldetag: 24.05.2002
(51) Int. Cl.: C09C 1/00

(54) **Optische Multischichtsysteme**

(30) Priorität: 12.06.2001 DE 10128488
(71) Anmelder: MERCK PATENT GmbH, 64293 Darmstadt (DE)
(72) Erfinder: Pfaff, Gerhard, Dr., 64839 Münster (DE); Andes, Stephanie, 63452 Hanau (DE); Fuchs-Pohl, Gerald, Dr., 64331 Weiterstadt (DE); Honeit, Ute, 64285 Darmstadt (DE)

(57) **Zusammenfassung**

Die vorliegende Erfindung betrifft optische Multischichtsysteme, umfassend Metallschichten, und mehrere Schichten, wobei diese Schichten mindestens ein Schichtpaket (A) aus einer farblosen dielektrischen Schicht aus einem Material mit einer Brechzahl n ≤ 1,8 und einer farblosen dielektrischen Schicht aus einem Material mit einer Brechzahl n > 1,8 sowie eine selektiv oder nicht selektiv absorbierende Schicht (B) umfassen, ein Verfahren zu deren Herstellung sowie deren Verwendung.

## Beschreibung

Die Erfindung betrifft optische Multischichtsysteme auf der Basis von Metallschichten und dielektrischen Schichten, ein Verfahren zu deren Herstellung sowie deren Verwendung.

Optische Multischichtsysteme mit Schichten aus reflektierenden Materialien, insbesondere Metallen, sind bekannt und finden breite Verwendung in vielen Bereichen der Technik, so zum Beispiel für Wertpapiere, für die Herstellung von Autolacken und dekorativen Beschichtungsmaterialien sowie zum Pigmentieren von Kunststoffen, Farben, Druckfarben, Papier, insbesondere für den Sicherheitsdruck, und dergleichen mehr.

In JP H7-759(A) wird ein mehrschichtiges Interferenzpigment mit metallischem Glanz offenbart, welches aus einem Substrat aus Aluminium-, Gold- oder Silberplättchen oder Plättchen aus Glimmer oder Glas, die mit Metallen beschichtet sind, und darauf befindlichen alternierenden Schichten aus Titandioxid und Siliziumdioxid besteht. Dieses Pigment besitzt ein hohes Deckvermögen. Der metallische Kern reflektiert das auftreffende Licht jedoch sehr stark, so dass der durch die Metalloxidschichten hervorgerufene Interferenzeffekt nur in sehr geringem Maße erkennbar ist und der harte metallische Glanz das Erscheinungsbild der Pigmente dominiert.

In US 4,434,010 sind optische Multischichtsysteme und Pigmente mit einer zentralen Schicht aus einem opaken, reflektierenden Material, zum Beispiel Aluminium, Gold, Kupfer oder Silber beschrieben, welche auf beiden Seiten mit einer ersten Schicht aus einem niedrig brechenden dielektrischen Material wie Siliziumdioxid, Magnesiumfluorid oder Aluminiumoxid und einer zweiten, semiopaken Metallschicht aus Chrom, Nickel oder Inconel beschichtet sind.
Diese Schichtsysteme und Pigmente werden vorrangig für Wertpapiere und den Druck von Wertpapieren eingesetzt und zeigen mit dem Betrachtungswinkel wechselnde Farben.

Optische Multischichtsysteme und daraus hergestellte Pigmente, die einen Farbverlauf aufweisen, sind in US 6,157,489 beschrieben. Diese besitzen eine zentrale Reflexionsschicht aus Aluminium, Silber, Kupfer und dergleichen, auf welcher beidseitig Schichten aus hochbrechenden dielektrischen Materialien wie beispielsweise Titandioxid, Zinksulfid oder Yttriumoxid und darauf eine Absorptionsschicht aus Chrom, Nickel, Palladium, Titan etc. aufgebracht sind.

Aus der DE 44 37 753 sind mehrfach beschichtete metallische Glanzpigmente bekannt, welche auf metallischen Substraten ein Schichtpaket aus
(A) einer farblosen Beschichtung mit einem Brechungsindex n ≤ 1,8 und
(B) einer selektiv absorbierenden Beschichtung mit einem Brechungsindex n ≥ 2,0
   sowie gewünschtenfalls zusätzlich
(C) eine äußere, farblose oder selektiv absorbierende, von der darunter liegenden Schicht (B) verschiedene Beschichtung aufweisen.

Dabei besteht die Schicht (A) beispielsweise aus Siliziumdioxid, Aluminiumoxid oder Magnesiumfluorid, während die Schicht (B) aus selektiv absorbierenden hochbrechenden Oxiden oder aus "eingefärbten" farblosen hochbrechenden Oxiden zusammengesetzt ist. Diese Pigmente sollen über interessante koloristische Eigenschaften verfügen und zur Erzeugung eines Farbflops, d.h. eines wechselnden farbigen Erscheinungsbildes in Abhängigkeit vom Betrachtungswinkel, geeignet sein.

Den aus den drei letztgenannten Veröffentlichungen bekannten optischen Multischichtsystemen und Pigmenten ist gemeinsam, dass die Interferenzfarbe der Pigmente im Wesentlichen durch die Brechzahl und die Dicke der ersten Schicht auf dem metallischen Substrat, die entweder eine niedrige oder eine hohe Brechzahl aufweist, sowie durch die Farbabsorption der darauf befindlichen Schicht bestimmt wird. Die Winkelabhängigkeit und die Farbintensität der Interferenzfarbe wird dagegen nur durch die Zusammensetzung und Dicke der ersten Schicht gesteuert. Es fehlt daher an Einflussmöglichkeiten, mit denen eine Feineinstellung der Farbintensität der Interferenzfarbe und /oder der Breite des Bereiches, in welchem ein winkelabhängiger Farbflop stattfindet, vorgenommen werden kann.

Es war daher die Aufgabe der Erfindung, optische Multischichtsysteme auf der Basis von Metallschichten und dielektrischen Schichten zur Verfügung zu stellen, welche über ein hohes Deckvermögen, eine hohe Farbintensität und/oder eine starke Winkelabhängigkeit der Interferenzfarbe über einen breiten Bereich verfügen und deren gewünschte Farbeigenschaften in einfacher Weise eingestellt werden können, sowie ein Verfahren zu deren Herstellung bereitzustellen und geeignete Verwendungsmöglichkeiten aufzuzeigen.

Diese Aufgabe wird erfindungsgemäß gelöst durch optische Multischichtsysteme, umfassend eine Metallschicht und darauf auf beiden Seiten oder auf einer Seite aufgebracht mehrere Schichten, umfassend
(A) mindestens ein Schichtpaket, bestehend aus
   i) einer farblosen dielektrischen Schicht aus einem Material mit einer Brechzahl n ≤ 1,8 und
   ii) einer farblosen dielektrischen Schicht aus einem Material mit einer Brechzahl n > 1,8, sowie
(B) eine selektiv oder nicht selektiv absorbierende Schicht,
wobei keine der Schichten (A) oder (B) die Metallschicht vollständig umhüllt.

Optional können die erfindungsgemäßen Glanzpigmente eine äußere Schicht (C) aufweisen.

Gegenstand der Erfindung ist ebenfalls ein Verfahren zur Herstellung der oben definierten optischen Schichtsysteme, in welchem auf einem Band Metallschichten, dielektrische Schichten und absorbierende Schichten aufeinander so abgeschieden werden, dass die Metallschicht auf einer Seite oder auf beiden Seiten mit den Schichten (A), (B) und optional (C) beschichtet wird.

Gegenstand der Erfindung ist zusätzlich auch die Verwendung der oben definierten Multischichtsysteme in Farben, Lacken, Druckfarben, Kunststoffen, kosmetischen Formulierungen, keramischen Materialien, Papier, Folien, Verpackungsmaterialien, Gläsern, Pigmentpräparationen, Trockenpräparaten, in Sicherheitsanwendungen und zur Lasermarkierung.

Unter optischen Multischichtsystemen werden mehrschichtige optische Filme und Pigmente verstanden, wobei letztere durch Zerkleinern aus den Filmen hergestellt werden.

Die Metallschicht ist lichtundurchlässig oder teilweise lichtdurchlässig und reflektierend und kann alle für Metalleffekte bekannten Metalle und Legierungen umfassen, so zum Beispiel Eisen, Stahl, insbesondere Edelstahl, Aluminium, Kupfer, Nickel, Chrom, Zink, Zinn, Silber, Gold, Platin, Kobalt, Lanthanide und Titan sowie Mischungen oder Legierungen aus zwei oder mehreren Metallen, wie Messing oder Bronzen.
Bevorzugt besteht die Metallschicht aus Aluminium.

Ist die Metallschicht teilweise lichtdurchlässig, hat sie eine Dicke von 5-35 nm. Ist sie lichtundurchlässig, hat sie eine Dicke von größer 35 nm.

Das Schichtpaket (A) besteht aus einer farblosen dielektrischen Schicht i) aus einem Material mit einer Brechzahl n ≤ 1,8 und einer farblosen dielektrischen Schicht ii) aus einem Material mit einer Brechzahl n > 1,8. Die Reihenfolge dieser Schichten ist nicht festgelegt und kann in Abhängigkeit von den gewünschten Farbeffekten bestimmt werden. Überraschenderweise wurde festgestellt, dass die Reihenfolge des Schichtauftrages die Farbeigenschaften des erfindungsgemäßen optischen Multischichtsystems wesentlich beeinflusst, obwohl die aus dem Stand der Technik bekannten Systeme, die entweder eine Schicht i) oder eine Schicht ii) bei ansonsten identischem Schichtaufbau aufweisen, im wesentlichen über miteinander vergleichbare optische Eigenschaften verfügen.

Wird auf der Metallschicht zuerst eine Schicht i) mit einer Brechzahl von n ≤ 1,8 und danach eine Schicht ii) mit einer Brechzahl von n > 1,8 aufgebracht, so lassen sich gegenüber dem Aufbringen von Einzelschichten, unabhängig von deren Brechzahl, bei ansonsten identischem Schichtaufbau eine Erhöhung der Intensität der Interferenzfarbe und/oder eine Verbreiterung des Farbbereiches, in dem ein Farbflop abhängig vom Betrachtungswinkel beobachtet werden kann, feststellen.

Wird dagegen auf der Metallschicht zuerst eine Schicht ii) mit einer Brechzahl von n > 1,8 und danach eine Schicht i) mit einer Brechzahl von n ≤ 1,8 aufgetragen, so lässt sich bei ansonsten identischem Schichtaufbau neben einer Erhöhung der Intensität der Interferenzfarbe auch ein Farbflop in Abhängigkeit vom Betrachtungswinkel einstellen, welcher nicht wie üblicherweise bei bekannten Systemen mit nur einer dielektrischen Schicht und ansonsten identischem Schichtaufbau, die aus dem Stand der Technik bekannt sind, einen Farbverlauf von einer Interferenzfarbe über alle denkbaren Zwischentöne zur nächsten Interferenzfarbe zeigt, sondern bei welchem sich zwei verschiedene Interferenzfarben über ein Unbunt bei der Änderung des Betrachtungswinkels miteinander abwechseln. Damit lässt sich zum Beispiel ein harter Farbwechsel von Purpur über Unbunt nach Grün einstellen.

Durch die Gestaltung der Reihenfolge der Schichten i) und ii) sowie über die Auswahl der Materialien für diese Schichten und die Bestimmung der individuellen Dicken der Schichten ergeben sich für den Fachmann daher eine Vielzahl von Möglichkeiten, optisch attraktive Multischichtsysteme für die verschiedensten Anwendungsbereiche gezielt herstellen zu können. Die dafür erforderlichen Einzelmaßnahmen sind dem Fachmann allgemein bekannt und erfordern kein erfinderisches Zutun.

Das Schichtpaket (A) ist in den erfindungsgemäßen optischen Multischichtsystemen ein- oder mehrfach, bevorzugt jedoch einfach vorhanden.

Die farblose dielektrische Schicht i) aus einem Material mit einer Brechzahl n ≤ 1,8 ist aus geeigneten Metallverbindungen wie Metalloxiden, Metallfluoriden oder deren Gemischen zusammengesetzt, die sich filmartig und dauerhaft aufbringen lassen.
Beispiele hierfür sind SiO₂, Al₂O₃, B₂O₃ oder MgF₂.
Bevorzugt sind SiO₂ und MgF₂ oder deren Gemische und besonders bevorzugt SiO₂.

Die Dicke dieser Schicht ist im allgemeinen 100 bis 1000 nm, vorzugsweise größer als 150 bis 600 nm.

Für die farblose dielektrische Schicht ii) aus einem Material mit einer Brechzahl n > 1,8 werden Metallverbindungen, vorzugsweise Metalloxide, Metallsulfide oder deren Gemische eingesetzt, beispielsweise TiO₂, ZrO₂, SiO, HfO₂, Y₂O₃, Ta₂O₅, ZnO, SnO₂, ZnS, bevorzugt jedoch TiO₂ und ZnS und insbesondere TiO₂.
Diese Schicht weist eine Dicke von 30 bis 500 nm und insbesondere von 200 bis 350 nm auf.

Die selektiv oder nichtselektiv absorbierende Schicht (B) ist hinsichtlich der Brechzahl des aufgebrachten Materials oder Materialgemisches nicht beschränkt und kann sowohl hochbrechende als auch niedrigbrechende Materialien umfassen. Sie ist jedoch mindestens teilweise lichtdurchlässig (semitransparent) und muss daher bezüglich ihrer Schichtdicke sorgfältig auf die verschiedenen eingesetzten Materialien abgestimmt werden.

Als Materialien kommen insbesondere Metalle wie beispielsweise Chrom, Wolfram, Kobalt, Nickel, Kupfer, Molybdän, Eisen, Silber, Gold, Palladium, Titan, Vanadium, Niob, Platin, aber auch Aluminium sowie Mischungen oder Legierungen aus zwei oder mehreren Metallen in Betracht. Ein Beispiel hierfür ist Inconel, eine Legierung aus 76 Gew.-% Nickel, 15 Gew.-% Chrom und 9 Gew.-% Eisen.

Ebenso geeignet sind jedoch auch Metalloxide, insbesondere solche, die von sich aus absorbierend sind, aber auch solche, die durch Einlagerung von oder Beschichtungen mit absorbierenden Materialien absorbierend gemacht werden können.
Besonders geeignete Metalloxide sind hierbei die verschiedenen Eisenoxide unterschiedlicher Modifikationen, Chrom(III)oxid, Titan(III)oxid und die bekannten farbigen Titansuboxide, Vanadiumdioxid, Vanadiumtrioxid oder auch Mischoxide sowie Mischungen.

Weiterhin kann die Schicht (B) durch nichtselektiv absorbierende Materialien gebildet werden. Beispiele hierfür sind Magnesiumfluorid oder Siliziummonoxid, die Chrom enthalten oder Titanmonoxid, das ebenfalls Chrom enthält.

Die Schichtdicke der Schicht (B) bestimmt sich aus dem eingesetzten Material und dem Erfordernis, dass diese Schicht für das sichtbare Licht mindestens noch teilweise durchlässig sein muss.

Für nichtselektiv absorbierende Materialien liegt die Dicke dieser Schicht bei etwa 5 bis 100 nm, wobei der untere Bereich von 5 bis 25 nm, insbesondere 5 bis 20 nm, für stark absorbierende Metalle wie Chrom und Molybdän ausreichend ist.

Werden dagegen selektiv absorbierende Metalloxide eingesetzt, kann die Dicke der Schicht (B) 5 bis 500 nm, vorzugsweise 10 bis 100 nm, betragen.

In der vorliegenden Erfindung besteht die Schicht (B) vorzugsweise aus Chrom mit einer Schichtdicke von 5 bis 20 nm, aus Fe₂O₃ mit einer Schichtdicke von 10 bis 100 nm oder aus Aluminium mit einer Schichtdicke von 5 bis 30 nm.

Durch die selektiv oder nicht selektiv absorbierende Schicht (B) wird die Reflexion des auftreffenden sichtbaren Lichtes an der Metallschicht abgeschwächt und der durch die Zwischenschichten i) und ii) eingestellte Farbeffekt verstärkt. Insbesondere bei der Einarbeitung der aus dem optischen Multischichtsystem erhaltenen Pigmente in die üblichen Farblacksysteme kommen daher die optischen Vorteile dieser Pigmente wie erhöhte Intensität der Interferenzfarben verbunden mit einem hohen Deckvermögen und metallischem Glanz, sowie die gezielt eingestellten erweiterten Farbbereiche für den Farbflop oder ein gewollter harter Farbwechsel von einer Farbe in eine andere ohne Zwischenfarbtöne gut zur Geltung.

Die optischen Multischichtsysteme können optional auch eine äußere Schicht (C) aufweisen. Diese soll vorzugsweise die darunter liegende Schicht (B) schützen und die Pigmente auf diese Weise stabilisieren.
Als Materialien für die äußere Schicht (C) können farblose oder selektiv absorbierende Metalloxide wie zum Beispiel SiO₂, Al₂O₃, TiO₂, ZrO₂, Fe₂O₃ oder auch Cr₂O₃, eingesetzt werden.
Liegt das Multischichtsystem in Form eines Pigmentes vor, kann eine nasschemische Nachbehandlung erfolgen, durch welche sowohl ihre chemische Stabilität erhöht als auch ihre Handhabung, insbesondere die Erleichterung des Einbringens in verschiedene Medien, verbessert wird. Dafür kommen insbesondere Verfahren in Frage, welche in DE 22 15 191, DE 31 51 354, DE 32 35 017, DE 33 34 598, DE 40 30 727, EP 0 649 886, WO 97/29059, WO 99/57204 oder US 5,759,255 beschrieben sind. Die Schicht (C) ist im allgemeinen etwa 1 bis 500 nm dick.

Die erfindungsgemäßen optischen Multischichtsysteme können zwischen der Metallschicht und dem Schichtpaket (A) und/oder zwischen dem Schichtpaket (A) und der Schicht (B) noch eine zusätzliche Schicht enthalten, welche aus Metalloxiden, Metallfluoriden, Metallnitriden oder deren Gemischen besteht.

Die einzelnen Schichten können nach bekannten Verfahren durch Sputtern von Metallen, beispielsweise von Aluminium oder Chrom oder von Legierungen, wie zum Beispiel Chrom-Nickel-Legierungen sowie von Metalloxiden, beispielsweise von Titanoxid, Siliciumoxid oder Indium-Zinn-Oxid oder durch thermisches Verdampfen von Metallen oder Metalloxiden hergestellt werden.

Im folgenden soll das Aufbringen der Schichten durch Aufdampfen näher beschrieben werden:

Für die Herstellung des Schichtsystems auf dem Substrat kann eine Bedampfungsanlage eingesetzt werden, die aus den üblichen Komponenten, wie Vakuumpumpsystem, Druckmess- und Steuereinheiten, Verdampfereinrichtungen, wie Widerstandsverdampfer oder Elektronenstrahlverdampfer, Vorrichtung zur Einstellung bestimmter Druckverhältnisse sowie einem Gaseinlass- und Regelsystem für reaktive Gase besteht.

Die Hochvakuumaufdampftechnik ist ausführlich beschrieben in Vakuum-Beschichtung, Bände 1-5; Herausgeber Frey, Kienel u. Löbl, VDI-Verlag 1995.

Das Aufbringen der Schichten durch Sputter-Verfahren erfolgt auf folgende Weise:

Beim Sputterverfahren oder bei der Kathodenzerstäubung wird zwischen dem Träger und dem Beschichtungsmaterial, das in Form von Platten (Target) vorliegt, eine Gasentladung (Plasma) gezündet. Das Beschichtungsmaterial wird durch energiereiche Ionen aus dem Plasma, z.B. Argonionen, beschossen und dadurch abgetragen bzw. zerstäubt. Die Atome oder Moleküle des zerstäubten Beschichtungsmaterials werden auf dem Substrat niedergeschlagen und bilden die gewünschte dünne Schicht.

Für Sputterverfahren eignen sich besonders Metalle oder Legierungen. Diese können mit vergleichsweise hohen Geschwindigkeiten, insbesondere im sogenannten DC-Magnetron-Verfahren, zerstäubt werden.
Verbindungen wie Oxide oder Suboxide oder Mischungen aus Oxiden können durch Einsatz des Hochfrequenz-Sputtern ebenfalls zerstäubt werden. Die chemische Zusammensetzung der Schichten wird durch die Zusammensetzung des Beschichtungsmaterials (Target) bestimmt. Sie kann aber auch durch Zusätze zum Gas, das das Plasma bildet, beeinflusst werden. Insbesondere werden Oxid- oder Nitridschichten durch Zusatz von Sauerstoff oder Stickstoff im Gasraum hergestellt.

Die Struktur der Schichten kann durch geeignete Maßnahmen, wie Beschuss der aufwachsenden Schichten durch Ionen aus dem Plasma, beeinflusst werden.

Das Sputterverfahren ist ebenfalls beschrieben in Vakuum-Beschichtung, Bände 1-5; Herausgeber Frey, Kienel und Löbl, VDI-Verlag 1995.

Für die Herstellung der erfindungsgemäßen optischen Multischichtsysteme eignen sich vorzugsweise kontinuierlich oder diskontinuierlich ablaufende PVD-Vakuumbandbeschichtungsverfahren, bei denen die einzelnen Schichten des Schichtsystems nacheinander abgeschieden werden. Auf diese Weise können symmetrische oder unsymmetrische Multischichtsysteme gemäß der vorliegenden Erfindung hergestellt werden. Für die Erzeugung des Multischichtsystems muss ein geeigneter bandförmiger Träger vorliegen. Dieser Träger besteht aus einem flexiblen Material, zum Beispiel einem Polyester, wie Polyethylenterephthalat. Dieser Träger wird in Abhängigkeit von der gewünschten Weiterverwendung des erfindungsgemäßen Multischichtsystems vorzugsweise mit einer in einem Lösungsmittel oder thermisch löslichen Ablöseschicht (release layer) beschichtet, wenn das Multischichtsystem vom Träger abgelöst und gegebenenfalls in Pigmentform weiterverwendet werden soll. Der Träger kann jedoch auch ohne weitere Vorbeschichtung sofort mit dem erfindungsgemäßen Multischichtsystem beschichtet werden, wenn an eine Verwendung gedacht ist, bei der das erfindungsgemäße Multischichtsystem in Form von definierten Flächen, beispielsweise streifenförmig, kreisförmig oder dergleichen verwendet werden soll. Ebenso besteht die Möglichkeit, den Träger sowohl mit einer Ablöseschicht als auch mit einer Haftschicht zu versehen, bevor das erfindungsgemäße Multischichtsystem abgeschieden wird. In diesem Falle kann das Multischichtsystem als Fläche vom Träger gelöst werden, um anschließend mittels der Haftschicht auf andere Materialien in Flächenform aufgebracht werden zu können.

Es versteht sich von selbst, dass zur Erzeugung eines symmetrischen Multischichtsystems der erfindungsgemäßen Art auf einem bandförmigen Träger zuerst die äußere Schicht des Systems, d.h. optional die Schicht (C), als erste Schicht auf das Band abgeschieden wird. Im Falle eines unsymmetrischen Multischichtsystems kann auch die Metallschicht als erste Schicht auf das Band abgeschieden werden.

Liegen die erfindungsgemäßen Multischichtsysteme als Pigmente vor, sind sie mit einer Vielzahl von Farbsystemen kompatibel, vorzugsweise aus dem Bereich der Lacke, Farben und Druckfarben. Weiterhin sind diese Pigmente auch in Kunststoffen, keramischen Materialien, Papier, Gläsern, für die Lasermarkierung von Papier und Kunststoffen, in Sicherheitsanwendungen, Folien und Verpackungsmaterialien, sowie für Anwendungen im Agrarbereich, z.B. für Gewächshausfolien, geeignet. Aufgrund ihrer hohen Farbkraft sind sie insbesondere auch in kosmetischen Formulierungen, zum Beispiel in der dekorativen Kosmetik, vorteilhaft einsetzbar. Sie eignen sich ebenso zur Herstellung von Pigmentpräparationen und Trockenpräparaten, wie zum Beispiel Granulaten, Chips, Pellets, Briketts, etc., welche insbesondere in Druckfarben und Lacken Verwendung finden.

Für die verschiedenen Anwendungszwecke sind die Multischichtsysteme in Pigmentform auch vorteilhaft in Abmischung mit handelsüblichen Farbstoffen und Pigmenten, beispielsweise organischen Farbstoffen, organischen Pigmenten oder anderen Pigmenten, wie z.B. transparenten und deckenden Weiß, Bunt- und Schwarzpigmenten sowie mit plättchenförmigen Eisenoxiden, organischen Pigmenten, holographischen Pigmenten, LCPs (Liquid Crystal Polymers), und herkömmlichen transparenten, bunten und schwarzen Glanzpigmenten auf der Basis von metalloxidbeschichteten Glimmer- und SiO₂-Plättchen, etc., einsetzbar. Die Multischichtpigmente können in jedem Verhältnis mit handelsüblichen Pigmenten, Bindemitteln und Füllstoffen gemischt werden.

Werden die erfindungsgemäßen Multischichtpigmente in flächiger Form eingesetzt, sind sie insbesondere für die Herstellung von, oder direkt als Folien und Verpackungsmaterialien geeignet. Darunter sollen insbesondere Kaltprägefolien, Heißprägefolien, Laminierfolien, dekorative Folien, Beschichtungsfolien, Schrumpffolien oder Teile derselben verstanden werden.
Eine besondere Bedeutung kommt auch der Anwendung in Sicherheitsanwendungen zu, zum Beispiel als Sicherheitsfäden oder -streifen für Geldscheine, Wertpapiere, Ausweise, Geldkarten, Ausweishüllen oder dergleichen.

Die erfindungsgemäßen optischen Multischichtsysteme weisen ein hohes Deckvermögen auf und zeigen intensive Interferenzfarben. Abhängig von der Reihenfolge der aufgebrachten Schichten sind Farbeffekte wie z.B. eine Verbreiterung des Farbbereiches, in dem abhängig vom Belichtungsoder Betrachtungswinkel Farbänderungen beobachtet werden können, oder aber ein harter Farbumschlag von einer Farbe in eine andere Farbe, ohne dass die üblichen Farbzwischentöne erkennbar sind, gezielt einstellbar.

Die vollständige Offenbarung aller vorstehend genannten Patentanmeldungen, Patente und Veröffentlichungen ist durch Bezugnahme in dieser Anmeldung enthalten.

Die nachfolgenden Beispiele sollen die Erfindung näher erläutern, ohne sie jedoch zu beschränken.

### Beispiele

### Beispiel 1

Ein optisches Multischichtsystem mit dem unten beschriebenen Schichtaufbau wird durch Aufdampfen auf eine Folie aus Polyethylenterephthalat hergestellt. Die Folie ist mit einer Ablöseschicht aus Stearin beschichtet. Das Aufdampfen erfolgt in einer Hochvakuum-Aufdampfanlage A700Q der Firma Leybold AG.

| Schichtaufbau des Pigmentes | | |
|---|---|---|
| **Schicht-Nr.** | **Material** | **Schichtdicke (nm)** |
| 1 | Cr | 5 |
| 2 | TiO₂ | 20 |
| 3 | SiO₂ | 200 |
| 4 | Al | 100 |
| 5 | SiO₂ | 200 |
| 6 | TiO₂ | 20 |
| 7 | Cr | 5 |

Das Schichtsystem wird mit Aceton von der Folie abgelöst, mit Aceton gewaschen, getrocknet und in einer Mörsermühle der Firma Netsch 30 min gemahlen. Man erhält ein Pigment mit einer mittleren Teilchengröße von 40 µm.

Das Pigment zeigt einen farbintensiven Goldton mit einem ausgeprägten Farbflop ins Blaugrün.

### Beispiel 2

Ein optisches Multischichtsystem mit dem unten beschriebenen Schichtaufbau wird durch Aufdampfen auf eine Folie aus
Polyethylenterephthalat hergestellt. Die Folie ist mit einer Ablöseschicht aus Stearin beschichtet. Das Aufdampfen erfolgt in einer Hochvakuum-Aufdampfanlage A700Q der Firma Leybold AG.

| Schichtaufbau des Pigmentes | | |
|---|---|---|
| **Schicht-Nr.** | **Material** | **Schichtdicke (nm)** |
| 1 | Cr | 5 |
| 2 | SiO₂ | 380 |
| 3 | TiO₂ | 130 |
| 4 | Al | 100 |
| 5 | TiO₂ | 130 |
| 6 | SiO₂ | 380 |
| 7 | Cr | 5 |

Das Schichtsystem wird mit Aceton von der Folie abgelöst, mit Aceton gewaschen, getrocknet und in einer Mörsermühle der Firma Netsch 30 min gemahlen. Man erhält ein Pigment mit einer mittleren Teilchengröße von 40 µm.

Das Pigment zeigt einen ausgeprägten Farbwechsel von einem kräftigen Purpur über Unbunt nach einem intensiven Grün.

## Patentansprüche

1. Optische Multischichtsysteme, umfassend eine Metallschicht und darauf auf beiden Seiten oder auf einer Seite aufgebracht mehrere Schichten, umfassend
(A) mindestens ein Schichtpaket, bestehend aus
i) einer farblosen dielektrischen Schicht aus einem Material mit einer Brechzahl n ≤ 1,8 und
ii) einer farblosen dielektrischen Schicht aus einem Material mit einer Brechzahl n > 1,8, sowie
(B) eine selektiv oder nichtselektiv absorbierende Schicht,
wobei keine der Schichten (A) oder (B) die Metallschicht vollständig umhüllt.

2. Multischichtsysteme gemäß Anspruch 1, die plättchenförmig vorliegen.

3. Multischichtsysteme gemäß Anspruch 1, die in Form eines Filmes vorliegen.

4. Multischichtsysteme gemäß einem oder mehreren der Ansprüche 1 bis 3, die zusätzlich eine äußere Schicht (C) aufweisen.

5. Multischichtsysteme gemäß einem oder mehreren der Ansprüche 1 bis 4, wobei die Metallschicht aus Metallen, Metalllegierungen oder deren Gemischen besteht.

6. Multischichtsysteme gemäß einem oder mehreren der Ansprüche 1 bis 5, wobei das Material mit einer Brechzahl n ≤ 1,8 für die Schicht i) ein Metalloxid, Metallfluorid oder ein Gemisch derselben ist.

7. Multischichtsysteme gemäß einem oder mehreren der Ansprüche 1 bis 6, wobei das Material mit einer Brechzahl n > 1,8 für die Schicht ii) ein Metalloxid oder Metallsulfid oder ein Gemisch derselben ist.

8. Multischichtsysteme gemäß einem oder mehreren der Ansprüche 1 bis 6, wobei die selektiv oder nicht selektiv absorbierende Schicht aus einem mindestens teilweise lichtdurchlässigen Metall oder einem selektiv absorbierenden Metalloxid oder aus Legierungen oder Gemischen derselben besteht.

9. Multischichtsysteme gemäß Anspruch 5, wobei die Metallschicht aus Eisen, Stahl, Edelstahl, Aluminium, Kupfer, Nickel, Chrom, Zink, Zinn, Silber, Gold, Platin, Kobalt, Lanthaniden, Titan, Mischungen oder Legierungen derselben besteht.

10. Multischichtsysteme gemäß Anspruch 6, wobei das Material mit einer Brechzahl n ≤ 1,8 für die Schicht i) SiO₂, Al₂O₃, B₂O₃ oder MgF₂ oder ein Gemisch derselben ist.

11. Multischichtsysteme gemäß Anspruch 10, wobei das Material mit einer Brechzahl n ≤ 1,8 für die Schicht i) SiO₂ oder MgF₂ oder ein Gemisch derselben ist.

12. Multischichtsysteme gemäß Anspruch 11, wobei das Material mit einer Brechzahl n ≤ 1,8 für die Schicht i) SiO₂ ist.

13. Multischichtsysteme gemäß Anspruch 7, wobei das Material mit einer Brechzahl n > 1,8 für die Schicht ii) TiO₂, ZrO₂, SiO, HfO₂, Y₂O₃, Ta₂O₅, ZnO, SnO₂ oder ZnS oder ein Gemisch aus zwei oder mehreren von diesen ist.

14. Multischichtsysteme gemäß Anspruch 13, wobei das Material mit einer Brechzahl n > 1,8 für die Schicht ii) TiO₂ oder ZnS ist.

15. Multischichtsysteme gemäß Anspruch 14, wobei das Material mit einer Brechzahl n > 1,8 für die Schicht ii) TiO₂ ist.

16. Multischichtsysteme gemäß Anspruch 8, wobei die selektiv oder nicht selektiv absorbierende Schicht (B) aus Chrom, Wolfram, Kobalt, Nickel, Kupfer, Molybdän, Aluminium oder Eisenoxid, Chrom(III)oxid, Titan(III)oxid, Titansuboxid, Vanadiumoxid oder aus Gemischen derselben oder aus Legierungen aus zwei oder mehreren Metallen besteht.

17. Multischichtsysteme gemäß Anspruch 16, wobei die selektiv oder nicht selektiv absorbierende Schicht (B) aus Chrom, Aluminium oder Eisenoxid besteht.

18. Multischichtsysteme gemäß einem oder mehreren der Ansprüche 1 bis 17, wobei die Schicht i) eine Schichtdicke von 100 bis 1000 nm aufweist.

19. Multischichtsysteme gemäß Anspruch 18, wobei die Schicht i) eine Schichtdicke von größer als 150 bis 600 nm aufweist.

20. Multischichtsysteme gemäß einem oder mehreren der Ansprüche 1 bis 19, wobei die Schicht ii) eine Schichtdicke von 30 bis 500 nm aufweist.

21. Multischichtsysteme gemäß Anspruch 20, wobei die Schicht ii) eine Schichtdicke von 200 bis 350 nm aufweist.

22. Multischichtsysteme gemäß einem oder mehreren der Ansprüche 1 bis 21, wobei das Schichtpaket (A) aus einer Schicht i) auf der Metallschicht und einer darauf aufgebrachten Schicht ii) besteht.

23. Multischichtsysteme gemäß einem oder mehreren der Ansprüche 1 bis 21, wobei das Schichtpaket (A) aus einer Schicht ii) auf der Metallschicht und einer darauf aufgebrachten Schicht i) besteht.

24. Verfahren zur Herstellung der Multischichtsysteme gemäß einem oder mehreren der Ansprüche 1-23, wobei auf einem flexiblen Träger Metallschichten, dielektrische Schichten und absorbierende Schichten so aufeinander abgeschieden werden, dass die Metallschicht auf einer Seite oder auf beiden Seiten mit den Schichten (A), (B) oder optional (C) beschichtet wird.

25. Verwendung der Multischichtsysteme gemäß einem oder mehreren der Ansprüche 1 bis 23 in Farben, Lacken, Druckfarben, Kunststoffen, kosmetischen Formulierungen, keramischen Materialien, Papier, Folien, Verpackungsmaterialien, Gläsern, zur Lasermarkierung, in Sicherheitsanwendungen sowie in Trockenpräparaten und Pigmentpräparationen.
